(19) Europäisches Patentamt
European Patent Office
Office européen des brevets

(11) **EP 4 402 911 B1**

(12) **EUROPEAN PATENT SPECIFICATION**

(45) Date of publication and mention
of the grant of the patent:
**30.07.2025 Bulletin 2025/31**

(21) Application number: **23703374.1**

(22) Date of filing: **30.01.2023**

(51) International Patent Classification (IPC):
*H04R 29/00* (2006.01)   *A61F 11/14* (2006.01)
*G01H 3/14* (2006.01)   *H04R 5/033* (2006.01)
*A61F 11/06* (2006.01)

(52) Cooperative Patent Classification (CPC):
**H04R 29/00; A61F 11/06; G01H 3/14; H04R 5/033;**
**H04R 2430/01**

(86) International application number:
**PCT/SE2023/050078**

(87) International publication number:
**WO 2023/146464 (03.08.2023 Gazette 2023/31)**

(54) **ADAPTIVE HEARING HEALTH CONTROL**

ADAPTIVE HÖRGESUNDHEITSSTEUERUNG

CONTRÔLE ADAPTATIF DE LA SANTÉ AUDITIVE

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**

(30) Priority: **31.01.2022 SE 2250090**

(43) Date of publication of application:
**24.07.2024 Bulletin 2024/30**

(73) Proprietor: **Audiodo AB (publ)**
**211 13 Malmö (SE)**

(72) Inventor: **LUNDBÄCK, Jonas**
**235 35 VELLINGE (SE)**

(74) Representative: **Ström & Gulliksson AB**
**P.O. Box 4188**
**203 13 Malmö (SE)**

(56) References cited:
**US-A1- 2008 015 463     US-A1- 2008 159 547**
**US-A1- 2016 316 306**

**Description**

**TECHNICAL FIELD**

The present invention relates to hearing health, and more precisely to control of a sound dose of a listener.

**BACKGROUND**

Access to audio in all its forms have increased greatly with the introduction of portable electronics equipment such as the Walkman® and later mobile phones. An audio book, a favorite song or an interesting podcast is always within reach. This, in combination with an increase in general environmental noise from industry, traffic etc. have led to an increased awareness of the deteriorating hearing health of a large portion of humanity.

The World Health Organization (WHO) has provided several studies and report stating the danger of listening at high volumes during an extended period of time. For instance, according to WHO, more than 1 billion young people, 12-35 years, are at risk for hearing loss due to recreational exposure to loud sound. WHO continues to estimate the overall annual cost of unaddressed hearing loss to 980 billion USD globally. WHO estimates that 50% of hearing loss can be prevented through public health measures. Some prevention strategies target individual lifestyle choices such as exposure to loud sounds and music or wearing protective equipment such as earplugs. This can be assisted through implementing audio standards for personal audio systems and devices.

As a result, the International Telecommunication Union (ITU) has issued recommendation ITU-T H.870 titled "Guidelines for safe listening devices/systems". Herein, it is described how a dose of sound, or sound dose, is calculated and compared to limits that indicate safe listening. Further guidelines have been issued by e.g. occupational safety and health administrations of different countries and regions and other national institutes for occupational safety and health.

In attempts to prevent ear damage, headphones for consumer electronics have been provided with a predetermined maximum output level. This blunt approach does not consider listening duration and a user can therefore risk hearing health issues in the long term by listening at a too high level during a too long period of time.

From the above, it is understood that there is room for improvements. Publications US 2016/316306 A1, US 2008/159547A1 and US 2008/015463A1 disclose devices and methods for recording and limiting sound exposure.

SUMMARY

An object of the present invention is to provide a new type of audio control which is improved over prior art and which eliminates or at least mitigates the drawbacks discussed above. More specifically, an object of the invention is to provide a system for monitoring a sound dose of a listener that is convenient for the listener and reduces the risk of injuring the hearing of the listener. These objects are achieved by the technique set forth in the appended independent claim with preferred embodiments defined in the dependent claims related thereto.

In a first aspect, a system for controlling a sound dose of a listener is presented. The system comprises a cloud server operatively connected to a remote storage. The remote storage is configured to store a plurality of audio profiles. Each audio profile comprises sound pressure data and a hearing profile associated with specific users of the system. The system further comprises one or more audio playback arrangements comprising a transducer, a controller, and a microphone. The microphone is arranged to measure a current sound pressure experienced by the listener. The one or more audio playback arrangements are operatively connected to the cloud server and configured to determine an identity of the listener. The system is configured to associate the determined identity with a specific user of the system. The cloud server and/or the controller of the one or more audio playback arrangements comprises controlling circuitry configured to cause obtaining of an audio profile comprising sound pressure data and a hearing profile for the listener and obtaining of a current sound pressure experienced by the listener. Based on the sound pressure data, the hearing profile of the listener and the current sound pressure, the controlling circuitry if further configured to cause estimating of a current estimated time period describing an estimated time until the sound dose of the listener exceeds a sound dose threshold, by frequency weighting the current sound pressure based on the hearing profile of the listener. Responsive to the current estimated time period being below a dose period associated with the sound dose threshold, based on the sound pressure data and the current sound pressure, the controlling circuitry is further configured to cause calculating of an updated sound pressure. for which a current updated time period, describing an estimated time until the sound dose of the listener exceeds the sound dose threshold, meets or exceeds the dose period. The controlling circuitry is further configured to cause providing of instructions for controlling the current sound pressure to meet the updated sound pressure.

In one variant, the sound pressure data of the audio profile comprises historic sound pressure data comprising previous sound pressure experienced by the listener. This is beneficial as it makes the estimation of the current estimated time period more accurate.

In one variant, the sound pressure data of the audio profile comprises an accumulated sound pressure experienced by the listener. This is beneficial as it enables the storing and sharing of accumulated sound pressure. The accumulated sound pressure obtained may e.g. be accumulated during times when the listener listened to other audio playback arrangements.

In one variant, the sound pressure data of the audio profile comprises a sound pressure trend experienced by the listener. This is beneficial as it makes the estimation of the current estimated time period more accurate.

In one variant, the current sound pressure is obtained from a microphone arranged at an ear of the listener. This is beneficial as the current sound pressure may be directly obtained without undue processing.

In one variant, estimating a current estimated time period describing the estimated time until the sound dose of the listener exceed the sound dose threshold comprises determining a prediction model. This is beneficial as it makes the estimation of the current estimated time period more accurate.

In one variant, the prediction model comprises an average model. This is beneficial as it makes the estimation of the current estimated time period more accurate.

In one variant, the prediction model comprises a Recurring Neural Network, RNN, model. This is beneficial as it makes the estimation of the current estimated time period more accurate.

In one variant, the controlling circuitry is further configured to cause updating the sound pressure data of the audio profile for the listener based on the current sound pressure. This is beneficial as it e.g. simplifies storing and sharing of an accumulated sound pressure.

In one variant, the controlling circuitry is configured to cause providing of instructions for controlling the current sound pressure for a left ear and/or a right ear of the listener and the audio profile comprises separate sound pressure data for each of the left ear and the right ear of the listener. This is beneficial as it makes the control of the sound dose more accurate.

In a second aspect, a method for controlling a sound dose of a listener is presented. The method comprises obtaining an audio profile comprising sound pressure data for the listener and obtaining a current sound pressure experienced by the listener. The method further comprises, based on the sound pressure data and the current sound pressure, estimating a current estimated time period describing an estimated time until the sound dose of the listener exceed a sound dose threshold. When the current estimated time period is below a dose period associated with the sound dose threshold, the method comprises, based on the sound pressure data and the current sound pressure, calculating an updated sound pressure for which a current updated time period, which describe an estimated time until the sound dose of the listener exceeds the sound dose threshold, meets or exceeds the dose period; and providing instructions for controlling the current sound pressure to meet the updated sound pressure.

In one variant, the sound pressure data of the audio profile comprises historic sound pressure data comprising previous sound pressure experienced by the listener. This is beneficial as it makes the estimation of the current estimated time period more accurate.

In one variant, the sound pressure data of the audio profile comprises an accumulated sound pressure experienced by the listener. This is beneficial as it enables the storing and sharing of accumulated sound pressure. The accumulated sound pressure obtained may e.g. be accumulated during times when the listener listened to other audio playback arrangements.

In one variant, the sound pressure data of the audio profile comprises a sound pressure trend experienced by the listener. This is beneficial as it makes the estimation of the current estimated time period more accurate.

In one variant, the current sound pressure is obtained from a microphone arranged at an ear of the listener. This is beneficial as the current sound pressure may be directly obtained without undue processing.

In one variant, the audio profile further comprises a hearing profile of the listener. In this variant, the current estimated time may further be based on the hearing profile of the listener by frequency weighting the current sound pressure based on the hearing profile of the listener. This is beneficial as the weighted current sound pressure will be the actual sound pressure the listener perceives. As an extreme example, if the listener is deaf at certain frequency bands, the sound pressure at these frequency bands would be substantially harmless to the listener.

In one variant, the estimating of a current estimated time period describing the estimated time until the sound dose of the listener exceed the sound dose threshold comprises determining a prediction model. This is beneficial as it makes the estimation of the current estimated time period more accurate.

In one variant, the prediction model comprises an average model. This is beneficial as it makes the estimation of the current estimated time period more accurate.

In one variant, the prediction model comprises a Recurring Neural Network, RNN, model. This is beneficial as it makes the estimation of the current estimated time period more accurate.

In one variant, the method further comprises updating the sound pressure data of the audio profile for the listener based on the current sound pressure. This is beneficial as it e.g. simplifies storing and sharing of an accumulated sound pressure.

In one variant, the audio profile is obtained from a remote storage. This is beneficial as it e.g. simplifies storing and sharing of the audio profile.

In one variant, the method is performed for a left ear and/or a right ear of the listener, and the audio profile comprises separate sound pressure data for each of the left ear and the right ear of the listener. The is beneficial as it makes the control of the sound dose more accurate.

In a third aspect, a controller for controlling an audio playback arrangement is presented. The audio playback arrangement comprises an audio transducer and a microphone arranged to measure a current sound pressure experienced by a listener of the audio playback arrangement. The controller comprises controlling circuitry configured to cause the obtaining of an audio profile comprising sound pressure data for the listener and the obtaining of a current sound pressure experienced by the listener. Based on the sound pressure data and the current sound pressure, the controller is configured to cause the estimating of a current estimated time period describing an estimated time until the sound dose of the listener exceed a sound dose threshold. Responsive to the current estimated time period being below a dose period associated with the sound dose threshold, the controlled is configured to cause, based on the sound pressure data and the current sound pressure, the calculating of an updated sound pressure for which a current updated time period, which describe an estimated time until the sound dose of the listener exceeds the sound dose threshold, meets or exceeds the dose period. The controller is further configured to cause the providing of instructions for controlling the current sound pressure to meet the updated sound pressure.

In a fourth aspect, an audio playback arrangement comprising the controller of the second aspect is presented.

In one variant, the audio playback arrangement further comprises an audio transducer and a microphone arranged to measure a current sound pressure experienced by a listener of the audio playback arrangement.

In one variant, the controller is configured to obtain an audio profile comprising sound pressure data for the listener and obtain a current sound pressure experienced by the listener. Based on the sound pressure data and the current sound pressure, the controller is configured to estimate a current estimated time period describing an estimated time until the sound dose of the listener exceed a sound dose threshold. When the current estimated time period is below a dose period associated with the sound dose threshold, the controller is configured to, based on the sound pressure data and the current sound pressure, calculate an updated sound pressure, for which a current updated time period, describing an estimated time until the sound dose of the listener exceeds the sound dose threshold, meets or exceeds the dose period. The controller is further configured to provide instructions for controlling the current sound pressure to meet the updated sound pressure.

In one variant, the controller is further configured to perform the method according to the first aspect.

In one variant, the controller is further configured to control the audio transducers to playback a source signal received by the controller.

In one variant, the controller is further configured to process the source signal to adjust an intelligibility of the source signal.

In one variant, the audio playback arrangement further comprises an external microphone and the intelligibility of the source signal is adjusted based on a background noise obtained from the external microphone.

In one variant, the audio profile further comprises a hearing profile describing hearing impairments of the listener and the controller is further configured to process the source signal based on the hearing profile. This is beneficial as the weighted current sound pressure will be the actual sound pressure the listener perceives. As an extreme example, if the listener is deaf at certain frequency bands, the sound pressure at these frequency bands would be substantially harmless to the listener.

In one variant, the controller is further configured to control the transducer based on the instructions for controlling the current sound pressure to meet the updated sound pressure.

In a fifth aspect, a system for controlling a sound dose of a listener is presented. The system comprises a cloud server operatively connected to a remote storage configured to store an audio profile comprising sound pressure data for the listener, one or more audio playback arrangements comprising a transducer, a controller, and a microphone arranged to measure a current sound pressure experienced by the listener. Wherein said one or more audio playback arrangements are operatively connected to the cloud server, and the cloud server and/or said one or more audio playback arrangements comprises the controller of the second aspect.

In variant, the remote storage is configured to store a plurality of audio profiles, each comprising sound pressure data associated with an individual user of the system. Said one or more audio playback arrangements are configured to determine an identity of the listener. The system is further configured to associate said identity with an individual user of the system. This is beneficial as audio playback arrangement may be shared between a plurality of listener and/or a listener may use different audio playback arrangements, still with an accurate control of the sound dose.

In a sixth aspect, a computer program product comprising a non-transitory computer readable medium is presented. The non-transitory computer readable medium have thereon a computer program comprising program instructions. The computer program being loadable into a controller and configured to cause execution of the method according to the first aspect when the computer program is run by the controller.

BRIEF DESCRIPTION OF THE DRAWINGS

Embodiments of the invention will be described in the following; references being made to the appended diagrammatical drawings which illustrate non-limiting examples of how the inventive concept can be reduced into practice.

Fig. 1 is a block view of embodiments of the present disclosure;
Figs. 2a-b is a time series plot of a sound dose according to embodiments of the present disclosure;
Figs. 3a-d are schematic views of audio playback arrangements according to embodiments of the present disclosure;
Fig. 4 is a block diagram of a system according to embodiments of the present disclosure;
Fig. 5 is a signaling diagram of a system according to embodiments of the present disclosure;
Fig. 6 is a time series plot of a sound dose according to embodiments of the present disclosure;
Fig. 7 is a block diagram of a method according to embodiments of the present disclosure;
Figs. 8a-e are schematic views of controllers according to embodiments of the present disclosure;
Fig. 9 is a schematic view of a computer program according to embodiments of the present disclosure; and
Figs. 10a-d are schematic views of a computer program according to embodiments of the present disclosure.

DETAILED DESCRIPTION OF EMBODIMENTS

Hereinafter, certain embodiments will be described more fully with reference to the accompanying drawings. The invention may, however, be embodied in many different forms and should not be construed as limited to the embodiments set forth herein; rather, these embodiments are provided by way of example so that this disclosure will be thorough and complete, and will fully convey the scope of the invention, such as it is defined in the appended claims, to those skilled in the art.

The term "coupled" is defined as connected, although not necessarily directly, and not necessarily mechanically. Two or more items that are "coupled" may be integral with each other. The terms "a" and "an" are defined as one or more unless this disclosure explicitly requires otherwise. The terms "substantially," "approximately," and "about" are defined as largely, but not necessarily wholly what is specified, as understood by a person of ordinary skill in the art. The terms "comprise" (and any form of comprise, such as "comprises" and "comprising"), "have" (and any form of have, such as "has" and "having"), "include" (and any form of include, such as "includes" and "including") and "contain" (and any form of contain, such as "contains" and "containing") are open-ended linking verbs. As a result, a method that "comprises," "has," "includes" or "contains" one or more steps possesses those one or more steps, but is not limited to possessing only those one or more steps.

Herein, the word playback system, playback device or playback volume is to mean playback in the sense of reproducing audio data received. The term is not to mean playback in the limiting form of playback of stored audio or media but it is to encompass all forms of media e.g. real time media, stored media, streamed media, progressively downloaded media etc. In addition to this, the term compensation is to mean compensation in its broadest form and can, in this disclosure, be seen as synonymous to the term processing. This is applicable not only when describing actions but also when the term is used to describe an object, e.g. a compensation filter.

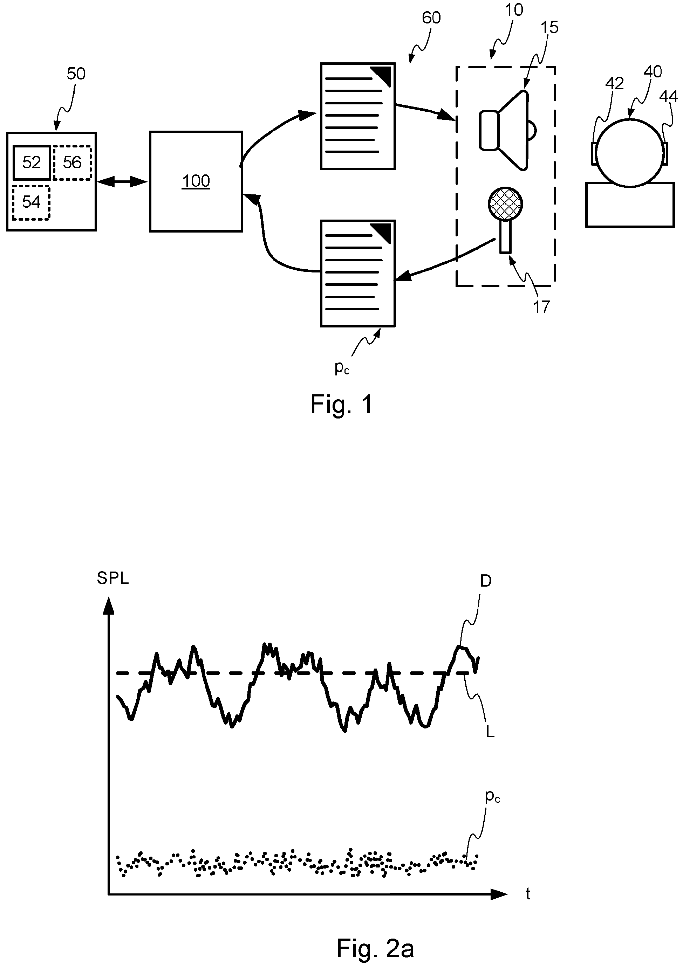

Starting from Fig. 1, the present disclosure is concern with controlling a sound dose $D$ experienced by a listener 40 such that the risk of a damage or injury to the hearing ability of the listener is reduced. The object is to achieve this without unduly limiting the listening experience and listening comfort of the listener 40. The sound dose $D$ is a measure of an accumulated sound pressure experienced by the listener for a specific duration of time. The sound dose $D$ may be calculated according to the previously cited ITU-T H.870 titled "Guidelines for safe listening devices/systems" as

$$D = \int_{t_1}^{t_2} \left( p_A(t) \right)^2 dt$$

where $p_A(t)$ is an A-weighted and diffuse-field corrected sound pressure experienced at time t. A-weighting is, as is commonly known, generally applied to instrument-measured sound pressure in an effort to account for the relative loudness perceived by the human ear. The sound dose $D$ as function of time may be referenced as a dose-profile. Sound pressure is commonly referenced to its logarithmic measure, sound pressure level (SPL). Sound pressure is a measured root mean square (rms) value and an internationally agreed reference $p_{ref}$ is $p_{ref} = 2 \cdot 10^{-5}$ N/m = 20 $\mu Pa$ and correspondingly in $dB$, $p_{ref,dB} = 20 \log_{10}(p_{ref}) \approx 93.9794$ dB SPL. Correspondingly, a sound pressure $p_a$ in Pascal has an

$SPL_a$ calculated as $SPL_a = 20 \cdot$ $$\log_{10} \left( {p_a}/{p_{ref}} \right) \ dB$$ .

It should be mentioned that the listener 40 referred to in the present disclosure is to mean a particular user currently using, i.e. listening to the audio playback arrangement 10.

The listener 40 is listening to an audio playback arrangement 10, which will be explained in further detail in other section of this disclosure, and being subjected to a current sound pressure $p_c$. At least part of the current sound pressure $p_c$ experienced by the listener 40 may be caused by a transducer device 15, transducer 15 for short, of the audio speaker arrangement 10. The current sound pressure $p_c$ is preferably obtained from one or more microphones 17 of the audio playback arrangement 10.

Preferably, the current sound pressure $p_c$ is an A-weighted sound pressure, but this is but one preferred embodiment. The A-weighted measure of sound pressure is an industry standard and limits and recommendations are typically communicated as A-weighted sound pressure, or if converted to SPL, as SPL presented in dBA. However, the skilled person will appreciate that the current sound pressure $p_c$ does not have to be an A-weighted sound pressure, but may very well be an unweighted sound pressure or a sound pressure weighted in any other suitable way. In the present disclosure, the sound pressure notation will be preferred, but the skilled person will understand that this is interchangeable with SPL as long as correct representation is used in respective calculations.

The current sound pressure $p_c$ is accumulated over time to calculate the sound dose $D$ of the listener. The sound dose $D$ is the current sound pressure $p_c$ accumulated during a dose period $T_D$ as illustrated in Fig. 2a exemplifying how samples of the current sound pressure $p_c$ are accumulated over time to the sound dose $D$. The sound dose D is described as a sliding average of the current sound pressure $p_c$ squared over the dose period $T_D$. In order to avoid discomfort, injury or damage to the haring of the listener 40, the sound dose $D$ should not exceed a sound dose threshold $L$, illustrated with a dashed line in Fig. 2a. The blunt force approach would be to simply control the audio playback arrangement 10 to stop providing audio to the transducer 15 thereby muting the audio from the audio playback arrangement 10 and reducing the current sound pressure $p_c$ to background sound. Such a muting would have to be in effect until the sound dose $D$ falls below the sound dose threshold $L$ and would greatly reduce the audio experience of the listener 40.

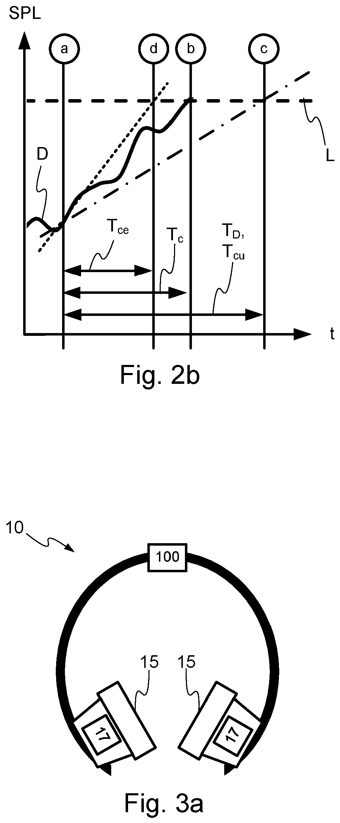

Fig. 2b is a partial view of the graph presented in Fig. 2a but with the current sound pressure $p_c$ removed. At a first time point $a$, the listener 40 increases the volume of the audio playback arrangement 10 and the current sound pressure $p_c$ increases. This causes the sound dose $D$, solid line of Fig. 2b, to increase and at a second time point $b$, after an actual current time period $T_c$, the sound dose $D$ exceeds the sound dose threshold $L$, dashed line of Fig. 2b. In the blunt force approach, the listener 40 would not be able to further listen to audio until the lapse of the dose period $T_D$ at a third time point c. However, based on the current sound pressure $p_c$ experienced by the listener 40 and the sound dose $D$ accumulated at the first time point $a$, it is, through the teachings of the present disclosure, possible to estimate a current estimated time period $T_{cu}$ describing an estimated time until the sound dose $D$ of the listener 40 exceed the sound dose threshold $L$. In Fig. 2b, the estimation is indicated by a dotted line that exceeds the dose threshold $L$ at a fourth time point $d$. As the current estimated time period $T_{ce}$ is shorter than the dose period $T_D$, the listener 40 will, assuming the current sound pressure $p_c$ is not reduced, be subjected to a sound dose $D$ that may injure the hearing of the listener 40. However, if the current sound pressure $p_c$ is reduced to an updated sound pressure $p_u$ such that a current updated time period $T_{cu}$ is the same as, or longer than the dose period $T_D$, the listener 40 will be able to enjoy audio at substantially the same volume as previously but without the risk of suffering from damaged hearing. In Fig. 2b, this is indicated by the dash-dotted line which crosses the sound dose threshold $L$ at the third point in time c. The current updated time period $T_{cu}$ is preferably calculated based on the updated sound pressure $p_u$ and current sound dose $D$.

Based on the explanation given with reference to Figs. 2a and b, some more details relating to Fig. 1 will be offered. In Fig. 1, an audio profile 50 associated with the listener 40 is preferably provided. The audio profile 50 comprises sound pressure data 52 for the listener 40. The sound pressure data 52 may comprise one or more accumulated sound pressures in the form a sound dose $D$. Additionally, the sound pressure data 52 may comprise a plurality of different sound doses $D_{1,2,...,n}$ accumulated during different dose periods $T_{D1,2...,n}$. The sound pressure data 52 may comprise data in the form of an accumulated sound dose $D$, i.e. a current sound dose $D$, but may additionally, or alternatively, comprise a series of previous sound pressure data experienced by the listener 40. The series of previous sound pressure data may be time stamped data or averaged data samples presenting an average sound pressure experienced by the listener 40 during certain time periods. The sound pressure data 52 may in other words be presented in many suitable ways and the present disclosure is not limited to the specific examples given above.

The audio profile 50 may further comprise a hearing profile 54 of the listener 40. The hearing profile 54 may be an audiogram describing the hearing abilities of the listener 40 at one or more frequencies. The hearing profile 54 may be based on a hearing test performed by the listener 40 and/or statistical data relating to e.g. age, gender, music taste etc.

Further to this, in some embodiments, the audio profile 50 further comprises a prediction model 56 for the listener 40. The prediction model 56 may be described as a sound dose-profile prediction 56 or a listening model 56. The prediction model 56 is a model that is usable to predict a listening behavior of the listener 40. The prediction model 56 will be explained in further detail elsewhere in this disclosure but may be formed in any suitable way. In some embodiments, the prediction model 56 comprise a time derivative of previous sound pressure data usable to estimate the current estimated time period $T_{ce}$. Additionally, or alternatively, the prediction model 56 may be an average model. Additionally, or alternatively, the prediction model 56 may be a Recurring Neural Network (RNN) model. The exemplified embodiments of the prediction model 56 may be combined to form e.g. a prediction model 56 comprising an average model and a RNN. It should further be mentioned that the prediction model 56 need not be comprised in the audio profile 50 but may be determined at any time by any of the devices of the present disclosure.

As explained, e.g. with reference to Fig. 2b, by using the audio profile 50 in general, and the sound pressure data

52 of the audio profile 50 in particular, in combination with the current sound pressure $p_c$, it is possible, as taught herein, to calculate the current estimated time period $T_{ce}$ describing the estimated time until the sound dose $D$ of the listener 40 exceed a sound dose threshold $L$.

More specifically, as illustrated in Fig. 1, a controller 100 is provided for controlling the sound dose $D$ experienced by the listener 40 of the audio playback arrangement 10. This may be accomplished by the controller 100 obtaining an audio profile 50 associated with a listener 40 and a current sound pressure $p_c$ from a microphone 17 of the audio playback arrangement 10. Based on the audio profile 50 and the current sound pressure $p_c$, the controller 100 may be configured to determine or estimate how long time, i.e. the previously presented current estimated time period $T_{ce,}$ the listener 40 may keep subjecting herself to the current sound pressure $p_c$ until the sound dose $D$ exceeds the sound dose threshold $L$. If the current estimated time period $T_{ce}$ is shorter than the dose period $T_D$, the controller 100 may be configured to cause the determining of an updated sound pressure $p_u$. The updated sound pressure $p_u$ is preferably configured such that a current updated time period $T_{cu}$ estimated based on the updated sound pressure $p_u$ will be substantially equal to, or longer, than the dose period $T_D$. The controller 100 may further be configured to provision instructions 60 for the audio playback arrangement 10 allowing the control of the current sound pressure $p_c$ to meet the updated sound pressure $p_u$.

It should be mentioned that the audio playback arrangement 10 mentioned with reference to Fig. 1 may be any suitable audio playback arrangement 10 and is not limited to the audio playback arrangements 10 listed herein. Preferably, the audio playback arrangement 10 comprises, or is operatively connected to, an audio transducer 15 and a microphone 17. The microphone 17, or microphones 17, are arranged to measure a current sound pressure $p_c$ experienced by a listener 40 of the audio playback arrangement 10.

In Figs. 3a-d, exemplary embodiments of audio playback arrangements 10 according to the embodiment of the present invention will be presented. These embodiments are non-limiting and the skilled person will appreciate, after reading this disclosure, that audio playback arrangements 10 in many other shapes, forms or configurations are possible and usable with the teachings of the present disclosure.

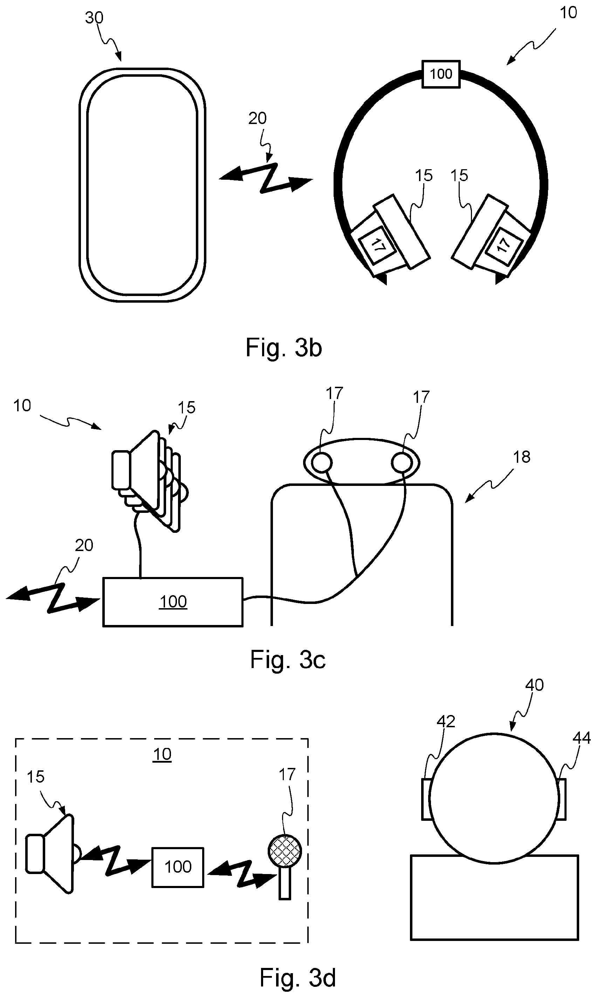

In Fig. 3a, an audio playback arrangement 10 in the form of a pair of headphones 10 is shown. The audio playback arrangement 10 comprises at least one audio transducer 15, or transducer 15 for short. The transducer 15 may be any form of a speaker 15 configured for transforming electrical signals to vibration detectable by a human ear 42, 44 (see Fig. 3d). The audio playback arrangement 10 generally comprises two transducers 15, but embodiments with only one transducer 15 or more than two transducers 15 are also feasible. The audio playback arrangement 10 further comprises one or more controllers 100. The controller 100 is configured for controlling the audio transducers 15 but may very well perform other control, processing and/or communication related tasks. The headphones 10 preferably comprises one or more microphones 17 arranged at an ear 42, 44 of a user of the headphones 10. The microphone 17 is configurable for measuring a current sound pressure $p_c$ at the ear 42, 44 of the listener 40. The microphone 17 may very well be configured to perform or assist in other functionality such as providing a noise reading at the ear 42,44 of the listener 40 usable for e.g. noise cancellation etc.

In Fig. 3b, another embodiment of the audio playback arrangement 10 is shown. Also in this embodiment, the audio playback arrangement 10 is a pair of headphones 10 comprising transducers 15, microphone(s) 17 for measuring the current sound pressure $p_c$ and a controller 100. In this embodiment, the controller 100 comprises, or is operatively connected to a transceiver (not shown) configured to communicate across an interface 20 with an electronic device 30. The electronic device may be any suitable rendering device such as a portable electronic device 30, preferably a mobile phone 30 as illustrated in Fig. 3b, a portable music player, a tablet, a laptop etc. The controller 100 may be configured to receive an audio stream across the interface 20. Alternatively, or additionally, the controller 100 may be configured to send and/or receive operational data across the interface 20. The operational data may be any data relating to the operation and/or control of the audio playback arrangement 10. The interface 20 may be a wired or a wireless interface 20 and may further be a digital or an analogue interface 20.

In Fig. 3c, another embodiment of the audio playback arrangement 10 is presented. In this embodiment, the transceiver(s) 35 are remote from the microphones 37 but the microphones 37 are still arranged for measuring the current sound pressure $p_c$ of the listener 40. In this particular embodiment, the microphones 17 are arranged in a headrest of a seat 18. The seat 18 may be a seat 18 of a vehicle, an office char, a lounge chair etc. In the particular embodiment shown in Fig. 3c, the seat 18 is a seat of a vehicle in the form of a car and the transducers 35 are the audio speakers of the car. The controller 100 may be a separate controller 100 or a controller 100 configured to perform other processing tasks of the car. The controller 100 may be provided with an interface 20 operatively connecting the audio playback arrangement 10 to e.g. a remote server (explained in other sections of the disclosure).

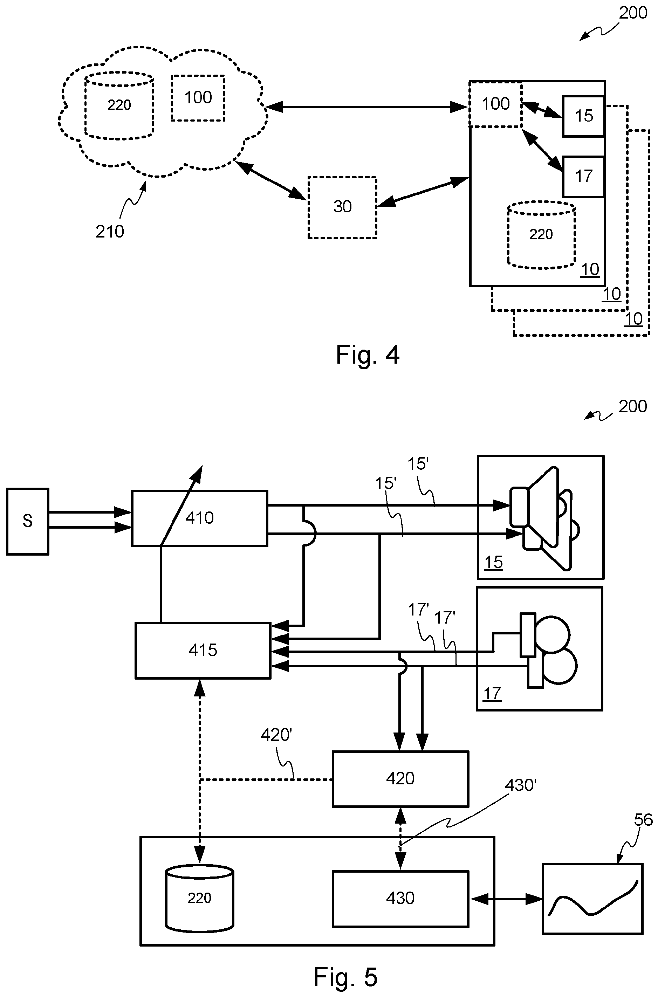

In Fig. 3d, yet another embodiment of the audio playback arrangement 10 is illustrated. In this embodiment, the transducer 15 and the microphone 17 are not physically connected to the controller 100, but arranged to be operatively connected to the controller 100. The microphone 17 is preferably located proximal to an ear 42, 44 of a listener 40 such that it may measuring the current sound pressure 70 at the ear 42, 44 of the listener 40. Each of the transducer 15 and the microphone 17 may be solitary devices, the transducer 15 may, for instance, be a Bluetooth speaker and/or a smart speaker, and the microphone 17 may be part of e.g. a hearing aid, a headset or a solitary microphone 17 configured to be worn in the proximity of the ear 42, 44 of the listened 40. The controller 100 may be a portable electronic device 30 such as a mobile phone configured to communicate with the microphone 17 and the transducer 15 directly through local connectivity (WIFI, Bluetooth, etc.) or indirectly through a cloud server. The controller 100 may be a connectivity gateway configured for providing e.g. smart home functionalities, voice control etc. In some embodiments, the controller 100 is comprised in the cloud server 210 (see Fig. 4).

As seen in Figs. 3a-d, the audio playback arrangement 10 may be arranged in numerous different ways and the embodiments illustrated may very well be combined or expanded in any way suitable. One common denominator between the audio playback arrangements 10 is that they comprise a microphone 17 configured for measuring the current sound pressure $p_c$ experienced by the listener 40. This may be accomplished by arranging the microphones 17 proximal to the ear 42, 44 of the listener 40. In some embodiments, e.g. where the audio playback arrangement 10 is a pair of in-ear headphones 10, the microphones 17 may be arranged in an ear cavity, or even an ear canal of the listener 40. It should be mentioned that the microphone(s) 17 may be very well be arranged distal from the listener 40 and configured, by e.g. directivity control, to measure the current sound pressure $p_c$ at the ear 42, 44 of a listener 40. The microphones 17 may optionally be an array of microphones configured to measure the current sound pressure 70 at the ear 42, 44 of a listener 40.

Embodiments of the present disclosure are generally described with the audio playback arrangement 10 configured to receive an audio stream from e.g. the portable electronic device 30. However, in some embodiments, the audio playback arrangement 10 may be configured for Hear-through mode in which a sound sensed by at least one of the microphones 17 per ear 42, 44 may be further enhanced according to the audio profile 50 and rendered by the headphone transducer 15.

Although most embodiments mention one sound dose $D$, one dose threshold $L$ and one dose period $T_D$, every embodiment may be extended to comprise more than one sound dose $D$, where each sound dose $D$ may be associated with a dose threshold $L$ and a dose period $T_D$.

With reference to Fig. 4, a system 200 for controlling the sound dose $D$ of a listener 40 will be described. The system 200 may comprise a cloud server 210 operatively connected to a storage 220 that may be a remote storage 220. The storage 220 may alternatively, or additionally, be partly or wholly arranged at the audio playback arrangement 10 and/or the portable electronic equipment 30 as presented herein. The storage 220 may be configured to store an audio profile 50 comprising sound pressure data 52 of the listener 40. The system 200 further comprises one or more audio playback arrangements 10, each comprising a transducer 15 and a microphone 17. Wherein the microphone 17 is arranged to measure a current sound pressure $p_c$ experienced by the listener 40. The system 200 further comprises one or more controllers 100 that may be distributed across all devices of the system 200 or be arranged at one or more of the devices of the system 200. In Fig. 4, the controller 100 is illustrated as being arranged at the cloud server 210 and at the audio playback arrangement 10. It should be understood that this is but one exemplifying embodiment and the controller 100 may further be stand alone or be partly or wholly arranged at the portable electronic device 30. Either way, the one or more audio playback arrangements 10 are operatively connected to the cloud server 210 and, if present, the portable electronic equipment 30. The one or more audio playback arrangements 10 may, in some embodiments, be operatively connected to the cloud server 210 via the portable electronic equipment 30.

Preferably, the remote storage 220 is configured to store audio profiles 50 of one or more listeners 40. The cloud server 210 may be configured to access and/or control the remote storage 220 to retrieve and/or store an audio profile associated with a particular listener 40. The cloud server may receive request from e.g. one or more devices of an audio playback arrangement 10 to provide the audio profile of the specific listener 40. The audio playback arrangement 10 may be configured to identify the listener 40 such that each particular listener is distinguished from other listeners 40. One such way is for the listener 40 having to identify himself before the audio playback arrangement 10 before being able to listen to audio. Alternatively, or additionally, the audio playback arrangement 10 may be configured to measure an acoustic footprint of e.g. the ear canal of the listener 40 and identify the listener 40 based on the acoustic footprint.

The configuration of a system 200 as described above is very beneficial as it allows several listeners 40 to share the same audio playback arrangement 10 and/or the same listener 40 to use different audio playback arrangements 10 and still ensure that a sound dose $D$ does not exceed the dose threshold $L$ for a given dose period $T_D$. To exemplify, a listener 40 may be enjoying music through a pair of headphones 10 which access an audio profile 50 of the listener via the cloud server 210. As listening progresses, the headphones 10 may share a current sound pressure $p_c$ and/or a sound dose D with the cloud server 210. The listener 40 may after some time change audio playback arrangement 10 to a different pair of headphones 10, to an audio playback arrangement 10 of a car (Fig. 3c) or to a home theater 10 (Fig. 3d). Either of the audio playback arrangements 10 may identify the listener 40 and access the audio profile 50 of the listener vi the cloud server 210, and based on the current sound pressure $p_c$ experienced by the listener, monitor and/or control the current sound pressure $p_c$ such that it does not exceed the dose threshold $L$. **In** other words, regardless of which audio playback arrangement 10 the listener 40 choses to use, the teachings of this disclosure enable the control of the sound dose $D$_such that it does not exceed the dose threshold $L$.

As is evident from the description of the system 200 in Fig. 4, the division into subcomponents, modules, devices and blocks is not fixed. **In** other words, one or more logical devices may be comprised in one or more physical devices. The logical division presented in Fig. 4 is provided for illustrative purposes and a person skilled in the art will be able to, after digesting the teachings of the present disclosure, to construct a number of different systems 200 with different layout but for equal purpose, i.e. for controlling the sound dose $D$ of a listener 40.

**In** one embodiment, the portable electronic equipment 30, in some embodiments referred to as a rendering device 30, store or receive audio data that is decoded and relayed to the controller 100. The rendering device 30 may further receive and transmit an audio profile 50 for the listener to a cloud server 210. The audio profile 50 comprises sound pressure data 52 of the listener 40, but may very well comprise further data relating to e.g. hearing and particularly hearing health of the listener 40. As previously mentioned, the rendering device 30 may be a portable electronic equipment 30, a mobile phone, a car stereo, or anything else with capabilities to connect to a remote location and receive and unpack and/or decode audio. The unpacked and/or decoded audio may be referenced as a source signal S (see Fig. 5) and may be a mono, a stereo or any other form of multi-channel signal.

The controller 100 may be configured to process audio streams, preferably a stream with the source signal S coming from the rendering device 30 together with microphone signals 17' (see Fig. 5) coming from the microphones 17. The controller 100 may process the source signal S using processing that is configured based on one or more of the microphone signals 17', data of the audio profile 50 and/or any other suitable processing parameters. Processing may be either mono or stereo. A processed source signal 15' (see Fig. 4) is sent to the transducer device 15. The processed source signal 15' may be in digital or analogue form and in the latter case, the transducer 15 is preferably configured to convert the digital audio signal 15' to an analogue audio signal and output the analog signal to one or more physical transducers 15. The transducers 15 may be mono e.g., Bluetooth speaker, or stereo e.g., a pair of headphones. In a car stereo or home theater, there may be numerous transducers 15.

The recording device 17 is comprised of at least one microphone 17 that, as previously indicated, preferably is located at an Ear Reference (ER) point i.e. located physically close the ear 42, 44 of the listener 40. In a pair of headphones 10, there are preferably at least two microphones 17, each one recording the audio at the left and right ER points, respectively.

The cloud server 210 of the system 200 in Fig. 4 may be referenced to as a server 210, a remote server 210 or a cloud service 210. The cloud server 210 may be configured to receive timely hearing health data, preferably comprised in the audio profile 50 for the listener 40. The hearing health data, or hearing data for short, comprised in the audio profile 50 may be computed based on usage by the listener 40, e.g. listening levels and duration. That is to say, the server 210 may be configured to receive a sound dose $D$, a current sound pressure $p_c$, and/or any other sound pressure data 52. The cloud service 210 may be configured to store all or some of the received hearing data associated with the listener 40 in the storage 220. The server 210 may further be configured to perform analysis on the hearing data and store a result of the analysis, preferably in the storage 220. The result of the analysis may be made accessible to the listener 40 such that the listener 40 may be able to access and view the hearing health, i.e. the audio profile 50, associated with the listener 40. The server 210 may be configured to provide updated hearing health models and sound dose $D$ budgets, i.e. how much accumulated sound pressure may the listener 40 be subjected to without exceeding the sound dose threshold $L$. All of these may be comprised in the audio profile 50 of the listener. The hearing health models, are preferably comprised in the audio profile 50, may be provided to the rendering device 30 and/or the audio playback arrangement 10 e.g. according to a predefined scheme or upon request from the rendering device 30 and/or the audio playback arrangement 10. A predefined scheme may be exemplified as e.g. at first connection of the day etc.

In Fig. 5, an exemplary signaling block diagram usable with any of the embodiments of e.g. the controller 100, the system 200 and/or the audio playback arrangement 10 of the present disclosure. The specific details and embodiments presented with reference to Fig. 5 are usable with any embodiment of the present disclosure and are not limited to this specific example. For the sake of explanation, and as a non-limiting example, assume that the block diagram of Fig. 5 is used on a system 200 where first frames of samples $N_p$ are transported from the rendering device 30 via a processing device, that may be the controller 100, to the transducer device 15. Further assume that second frames of samples $N_r$ are transported from the recording device 17 to a processing block 410.

A sample rate of various signals in the system 200 and frame sizes $N_p$ and $N_r$ may vary depending on e.g., hardware capabilities, signal quality and/or power consumption requirements. Using well-known resampling technics based on e.g., anti-aliasing filtering and sinc-interpolation, sample rate alignment and sample alignment may be readily obtained. Assume, without loss of generality, that $N_p = N_r$. Both are typical operations of sample alignment and resampling operation.

The system 200 in Fig. 2 is depicted using two transducers 15 and two microphones 17. Assume that the system 200 comprises an audio playback arrangement 10 as illustrated in Fig. 3b. The system 200 is assumed to be configured for stereo or two channels playback. One channel, or mono, playback is a special case where the source signal S is split into two identical stereo channels before being provided to the transducers 15.

At the top of Fig. 5, the source signal S may be received from e.g. the rendering device 30 (not illustrated in Fig. 5) and processed by the enhancements in the processing block 410. The enhancement may be time and frequency adaptive and may be controlled by a processing control block 415. Once the source signal S has been enhanced, the resulting audio signals 15' are relayed to the transducer 15 for playback.

Each channel of the source signal S may be processed individually. The processed source signal may be relayed to one or more transducers 15 e.g., in the case of a pair of headphones 10, a mono source signal S is split into two channels that are processed individually and rendered on the corresponding left and right headphone transducers 15. The corresponding is true for transducers 15 located near each ear 42, 44 e.g. in the seat 18 of a car.

At a point in the system where no further processing will be applied to the digital transducer signals 15', i.e. when only DA conversion remains, the transducer signals 15' may be provided to the processing control block 415 as a signal reference. The signal reference is usable for signal separation using e.g. linear echo cancellation.

The microphones 15, preferably at least one microphone 17 per ear 42, 44, senses (e.g. records, measures, etc.) the sound that reaches each ear 42, 44. For headphones 10 using active noise control (ANC), a microphone 17 is generally perfectly located in the ear-headphone cavity, next to the transducer 15. In a car, the preferred placement of each microphone would be in the head support of the seat 18, preferably with one microphone 17 at each side of a head of the listener 40.

Each microphone signal 17' represents a signal describing a sound that one ear 42, 44 have received. Using the microphone signals 17', the sound dose $D$, as described elsewhere in this disclosure, is calculated at the sound dose calculation block 420, preferably separately for each ear 42, 44. The calculated sound dose $D$ is stored. The sound dose $D$ may be stored locally at a local storage 220 of the audio playback device 10 and/or the rendering device 30. Preferably, the calculated sound dose $D$ is additionally stored (i.e. backed up, mirrored, duplicated etc.) at the remote storage 220 operatively connected to the server 210 or cloud service 210. The calculated sound dose D may be transferred to the cloud service 210 by means of the rendering device 30, i.e. the portable electronic equipment 30. As previously mentioned, the sound dose D may be calculated for a number of different dose periods $T_D$. The rate of the sampling of the microphone signal 17', the amount of storage available, the available bandwidth for communicating sound doses $D$ etc. may be useful in determining a minimum period $T_m$ during which the sound dose is preferably calculated for correct and accurate controlling of the enhancement of the source signal S. An exemplary and non-limiting setting for the minimum period $T_m$ is 1 minute.

In one embodiment, the sound dose D calculations are done per audio frame and aggregated to form a minimum sound dose $D_m$ per minimum period $T_m$. The sound doses $D$ for other dose periods $T_D$ are calculated as a sum of a number of minimum sound doses $D_m$. With a dose period $T_D$ of one hour and the minimum period $T_m$ being one minute a sound dose per hour $D_h = \sum_{m=1}^{60} D_m$. Consequently, the accumulated sound dose D is monotonically increasing.

According to the previously mentioned ITU-T H.870 titled "Guidelines for safe listening devices/systems", a recommended sound dose $D_R$ is given with a dose period $T_D$ of seven days. For adults, the recommended sound dose per week $D_{R7}$ (seven days) may be calculated as $D_{R7}$ = 1.6 Pa$^2$. This is commonly known as the reference dose. The reference dose $D_{R7}$ corresponds to an average SPL of 80 dBA. This is equivalent to a sound pressure of $10^{\left(\frac{80-\mathrm{p_{ref,dB}}}{20}\right)} = 0{,}2$ Pa and a sound pressure square equal to $10^{\left(\frac{80-\mathrm{p_{0dB}}}{20}\right)^2} = 0{,}04$ Pa$^2$ during 40 hours of listening which yields $D_{R7}$ = $10^{\left(\frac{80-93.9794}{20}\right)^2} \cdot 40 = 1.6$ Pa$^2$. The skilled person will, after reading this disclosure, appreciate that the sound dose $D$ and dose period $T_D$ may be set appropriately for each application and is not limited to the example given above.

A sound dose control block 430 may be configured to, at specific times e.g., periodically or at request from e.g. the rendering device 30 or the storage 220, upload the sound dose $D$ or sound does $D$ to the server 210 or cloud service 210. The sound dose control block 430 may further be configured to requests, determine and/or directly receive a model for a sound dose-profile prediction 56, i.e. the prediction model 56. This model 56 may, as previously explained, be comprised in the audio profile 50 and may be determined on historic and/or current sound pressure data 52 for the listener 40. The sound dose control block 430 may further be configured to requests, determine and/or directly receive an updated prediction model 56.

Using the prediction model 56, the sound dose control block 430 may be configured to check if the current accumulated sound dose $D$ during a specific dose period $T_D$ will lead to the exceeding of the associated dose threshold $L$. This predicts if the dose threshold $L$, i.e. the recommended maximum dose, will be exceeded at the end of the dose period $T_D$. Or as previously described, if the current estimated time period $T_{ce}$ is shorter than the dose period $T_D$. If the prediction concludes that the dose threshold $L$ is not exceeded ($T_{ce} > T_D$), no action is needed. If the prediction result in dose threshold $L$ being exceeded ($T_{ce} < T_D$), an action to decrease the current sound pressure may be $p_c$ is taken.

The functions, blocks and features described herein relating to e.g. processing, control, calculating etc. may be implemented in hardware, software or combinations thereof. The processing block 410, the processing control block 415, sound dose calculation block 420, sound dose control block 430 and/or the storage 220 may be comprised in the controller 100. The blocks may form part of a distributed system, alternatively or additionally, some functions or blocks may be fully or partly comprised in the controller 100.

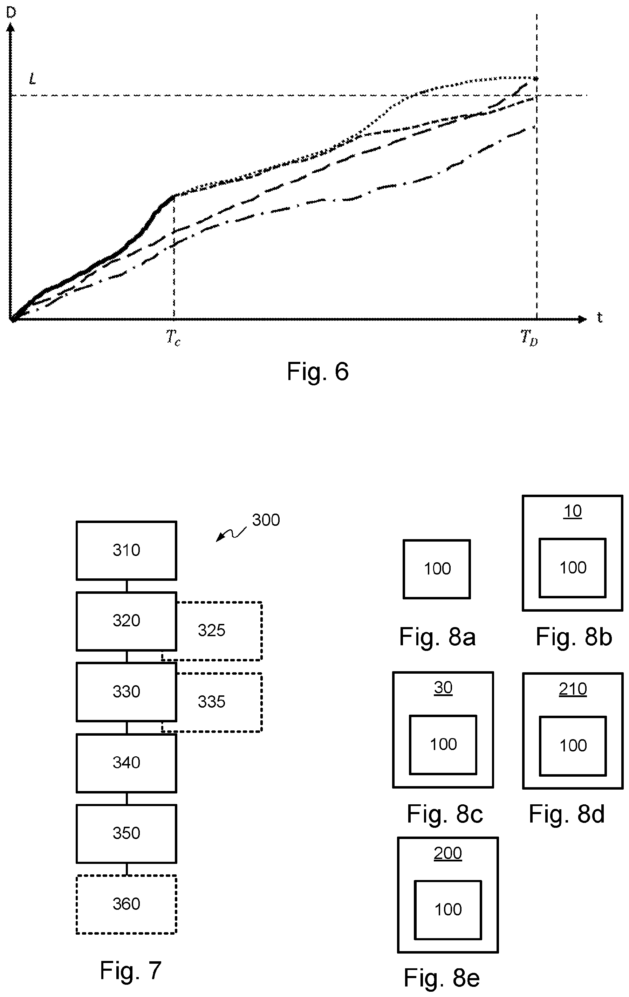

Fig. 6 depict a non-limiting example presented for further disclosure of the workings of embodiments of the present disclosure. In Fig. 6, the dose period $T_D$ is 168 hours (7 days) and the maximum recommended dose, i.e. the dose threshold $L$, during the dose period $T_D$ is, 1.6 $Pa^2h$. The lower line-dotted curve of Fig. 6 depicts a dose-profile during the dose period $T_D$. As seen, this line-dotted curve It does not reach the maximum limit dose threshold $L$. The long-lined curve, above the line-dotted curve, is an example of a listening behaviour causing the sound dose D to exceed the dose threshold $L$ before the dose period $T_D$ has lapsed. This may in the long run lead to hearing deficiency.

With continued reference to Fig. 6, to exemplify the prediction of the sound dose D, the solid curve corresponds to a listening behavior that is evaluated at the current time $Tc$ by a prediction algorithm according to teachings of the present disclosure. The outcome of the evaluation is the top dotted curve in Fig. 6. The estimated sound dose D based of the evaluation at the current time $T_c$ clearly exceeds the dose threshold $L$ before the end of the dose period $T_D$ ($T_{ce} < T_D$). Responsive to this, an adaptive frequency transfer function of the processing block 410 is adjusted, i.e. a new estimated sound dose D is calculated based on a the adjusted frequency transfer function and the resulting listening profile is shown in the short-lined curve of Fig. 6. As seen in Fig. 6, the adjustment is such that the short-lined curve does not exceed the dose threshold $L$ within the dose period $T_D$ ($T_{ce} > T_D$). Consequently, the risk of damaged hearing of the listener 40 is reduced. The frequency transfer function may further be adjusted to maximize an intelligibility of an audio stream given e.g. background noise, user hearing impairment etc.

It should be mentioned that the adjusted frequency transfer function may comprise nothing more than an attenuation across all frequencies effectively reducing the current sound *pressure* $p_c$ experienced by the listener 40. Alternatively, the processing block 410 may be configured to calculate the appropriate adaptive processing, i.e. enhancement, based on the source signal S, background noise, hearing impairments of the listener 40, accumulated sound dose D, and/or processing parameters. The processing parameters may include but are not limited to, an audiogram describing the listener's 40 hearing capabilities and/or an intelligibility mode. The processing parameters may be comprised in the audio profile 50 for the listener 40. An intelligibility mode parameter may be configured to indicate which intelligibility measure to be improved, the intelligibility measures are presented in more detail elsewhere in the present disclosure.

In the following, some additional implementation details, usable with any of the embodiments presented herein will be given. Assuming a standard calibration of the microphones 17, the current sound pressure $p_c$ may be calculated based a signal from the microphone(s) 17. The sound dose $D_m$ during the dose time $T_m$ may consequently be calculated as

$$D_m = T_m \frac{1}{N_m} \sum_{n=1}^{N_m} 0.5(z_L(n) + z_R(n))$$

where $f_s$ is the sampling frequency of the microphone signal 17' and $N_m$ = $T_m f_s$. The signals $z_L(n)$ and $z_R(n)$ are the microphones signals 17' corresponding to left and right ear 42, 44 after being transformed (standard microphone calibration and linear frequency weighting) into A-weighted sound pressure levels. As the microphones 17 are typically not placed at an eardrum of the listener 40, no diffuse field correction is generally needed. The sound dose D during the dose period $T_D$ is accumulated as $D_{Ack} = D_{Ack} + D_m$ for each minimum period $T_m$. The sound dose D as a function of time is monotonically increasing.

The prediction of the sound dose-profile during the dose period $T_D$ may be based on one or more of historical sound dose data, a feature set e.g., time, date, volume index and device identification, the current and past values of the sound dose D of the current dose period $T_D$ and combinations and equivalents of these.

One exemplary predictor is an average predictor that is constructed from the average of several previous dose-profiles i.e., $D(k) = AVG_h(D_h(k))$ where $D_h(k)$ is one out of H previous sound dose-profiles, $h = 1, ... , H$. The index k is the calculation (time) index corresponding to a unique time interval $T_m$ in a respective dose-profile, $k = 1, ... , K$. K is the last calculation index during the dose period $T_D$ and corresponds to $T_D$. $AVG_h(*)$ is the average operator.

A second exemplary predictor is a maximum based predictor $\hat{D}(k) = \max_{h}(D_h(k))$ .

In one embodiment using the predictor, the sound dose D at the end of the dose period $T_D$ may be estimated as $\hat{D}(K, D_{ack})$.

A third exemplary predictor is a regression model. The regression model may be a recurrent neural network (RNN), a Markow Chain or an auto regressive process. The RNN may be based on Long Short-Term Memory (LSTM) or Gated Recurring Units (GRU). The RNN using LSTM are suitable for multivariate multistep time-series prediction.

In general, a vector and an output vector of the RNN may be selected in numerous ways and the selection of the two, the size of the RNN along with the training data quality and the training of RNN will determine the performance of the RNN and its ability to output the desired output data given the input (feature) data.

The feature set may, as stated above, comprise past and present values of sound doses D during a current dose period $T_D$, time and date, volume index and device identification, e.g. an identifier of that audio arrangement 10. The input of the sound dose $D$ is beneficial for the workings of the prediction model, further to this time and date may impact a dynamic behavior since a sound dose $D$ increase may be time dependent e.g., only measured during office hours, at weekends etc.

Further to this, prolonged exposure to sound may cause the hearing of the listener 40 to be saturated and/or temporarily be worsened. As a consequence, the listener 40 may be inclined to increase the volume of the audio playback arrangement 10. Additionally, depending on the time, the listener 40 may be located at various places with more or less background noise e.g. visiting a loud cafeteria during lunch etc. Hence, using one or more of time, day and/or volume index to predict the dose-profile is beneficial.

The predicted dose-profile $\hat{D}(k)$, may, in some embodiments, be usable in providing a gain $G(k) = f(\hat{D}(K, D_{ack}), k)$ such that if $G(k)$ is applied to the source signal $S$ in the processing block 410, then $\max_k \widehat{D}(k) < D_{max}$ and since the sound dose $D$ is monotonically increasing, $\hat{D}(K) < D_{max}$, i.e. the, the source signal $S$ is attenuated (an attenuation is represented by $G(k) < 1$). In some embodiments, the gain $G(k)$ may be a frequency dependent gain $G(k, f)$ and the source signal $S$ may be increased at some frequencies and attenuates at others still fulfilling the above listed criteria.

The calculation of the gain $G(k)$ may be updated at regular intervals e.g., every minimum period $T_m$. Considerations regarding the gain $G(k)$ may comprise e.g. an update rate versus a stability of a frequency transformation of the source signal $S$. A comparably fast and large variation of gain on an audio signal may cause audio quality degradations.

In some embodiments, the gain $G(k) = \dfrac{D_{max}}{\hat{D}(K) - \hat{D}(k)} - D_{Ack}(k)$ may be configured to effectively decrease the sound dose D if applied during a remainder of a dose period $T_D$ i.e. $T_D - T_c$, wherein $T_c$ is the current time (see Fig. 6).

$$L_{spl} = 20 \cdot log_{10}\left(\sqrt{G(k) * D_{Ack}(k)/T_m}\middle/ p_{ref}\right)$$

This may be used to calculate a maximum SPL as during the next minimum period $T_m$, which reference the gain to the current sound dose $D$. Consequently, $L_{spl}$ is the maximum SPL obtained from the sound dose calculation.

Additional embodiment for the gain $G(k)$ and subsequent maximum SPL $L_{spl}$ may comprise e.g. rules describing when to apply the gain $G(k)$ resulting in a decreased sound pressure or not. In some embodiments, the limitations to the current sound pressure $p_c$ may only be invoked if the sound dose $D$ is above a limit threshold that lies between the current sound pressure $p_c$ and the sound dose threshold $L$. In further embodiment, the controller 100 may be configured to frequency dependently limit the current sound pressure $p_c$ until the limit threshold is exceeded. This may comprise decreasing the current sound pressure $p_c$ by attenuating higher frequency content etc. of the source signal S. For a person skilled in the art, a set of rules that control the sound pressure limitation may be readily established after digesting the teachings of the present disclosure.

In some embodiments, the sound dose-profile prediction 56 accounts for the listener's 40 behavior during the dose period $T_D$. In these embodiments, decreasing the current sound pressure $p_c$ according to the teachings herein may be executed at some time index k where the listener 40 typically is subjected to a higher average sound pressure. Additionally, or alternatively, the decrease of the current sound pressure $p_c$ may be performed by adjusting a frequency weighting of the source signal S such that non-important frequency content and/or frequency content not heard by the listener 40 is removed or attenuated. Consequently, non-important frequency content and frequency content outside what the listener 40 can hear, will no longer contribute to the sound dose $D$. By doing this, the risk of the sound dose $D$ exceeding the dose threshold $L$ and thereby causing damage to the hearing of the listener 40 is reduced without compromise of the listener's 40 experience of the audio.

As indicated in Fig. 5, The SDC 430, which may be comprised in the controller, preferably performs sound dose-profile prediction 56 and assert that the prediction model 56 is correct. As data is sent to the server 210 or cloud service 210, the sound dose-profile prediction 56 may be updated remotely and downloaded to the SDC 430 via the rendering device 30. Depending on the type of prediction model 56, the update or construction of the prediction model 56 may additionally, or alternatively, be done locally e.g., by the processing block 430 or the rendering device 30. An average prediction model as exemplified above may be recursively updated and require very little processing and storage when updated locally. An RNN prediction model 56 generally require more resources and is preferably updated remotely. A prediction model update may be triggered e.g. on a regular basis with set time intervals or when a set stability condition is failed. The predictor model 56 is preferably associated with the listener 40 and consequently changes if the listener 40 changes.

In the following further implementation details, usable with any of the embodiments presented herein will be given. As mentioned elsewhere in the present disclosure, the server 210 or cloud service 210 may be configured for remote storage of user data and sound dose data, i.e. the audio profile 50 of the listener 40. The prediction model 56 may be updated and/or retrieved by the portable electronic equipment 30. The prediction model 56 may be sent as parameters e.g. the parameters of the RNN or the Auto-regressive process. The server 210 or cloud service 210 may, in some embodiments, be described as part of the system 200 for providing storage and/or calculation of prediction models 56.

The processing control block 415 may, for each microphone signal 17', estimate the stability of an acoustic environment using the transducer signal 15' and the microphone signals 17'. This may be accomplished in many ways. In one embodiment, this is done by evaluating a short-time power spectrum change per time unit per frequency band. In another embodiment, this is done by comparing an SPL averaged during a short time period to an SPL averaged during a comparably longer time period, by which equal quantities indicate at least a semi-stationary acoustic environment.

The processing control block 415 may further apply a hearing model, preferably the hearing profile 54 of the listener 40, on the microphone signal 17' to model how the listener 40 would have perceived the sound. Parameters enhancing the source signal S may be calculated based on the perceived sound and/or the hearing model 54.

The microphone signals 17' are converted from time-domain into frequency domain preferably by discrete frequency transform (DFT), i.e. using an FFT implementation of the DFT. For each 1/3 octave frequency band, the adjacent frequency bins of the DFT are amplitude root mean squared accumulated such that a resulting 1/3 octave frequency representation is obtained for all signals.

Estimation of an acoustics propagation may be performed in several ways. In some embodiments, the transducer signal 15' is inputted to a linear echo cancellation function based on e.g., Least Mean Square (LMS) adaptation. This may be usable to estimate an echo path i.e., the acoustic path between transducer 15 and microphone 17. In some embodiments, the acoustic path is estimated as a secondary path as used in an ANC application. In some embodiments, the acoustic path is measured using acoustic measurement equipment, modelled as a frequency function or a filter, and stored for usage during operation. The latter is a simpler construction compared to linear echo cancellation or ANC, but less flexible and may require one model for each acoustic path considered under normal operation.

To estimate the background noise, the microphone signal 17' may, in some embodiments, be split into a propagated source signal (echo) and a background noise via signal separation. Signal separation may be performed using e.g. an echo controller to remove the propagated source signal, using signal-subspace division e.g. the Karhunen-Loeve transform which is suitable since the covariance matrix of the source signal is known and the approximate echo path from the acoustic propagation calculations.

In some embodiments, the background noise may be measured as the microphone signal 17' when the source signal S is zero, or when the source signal S is less than a set threshold. If the source signal S is speech, there are natural pauses in the speech signal, while in music the same may not be true. For the case of music, the estimation of the background noise is preferably performed by removing the propagated source signal S from the microphone signal 17'. The latter feature is, although more resource demanding than using pauses in the source signal S, also usable on speech signals.

The hearing model 54 is preferably configured to take the microphone signals 17' as input. The microphone signals 17' may be converted into equivalent sound pressure values using e.g. the conversion function from the calculation of the sound dose D. Then, a frequency and level dependent gain may be calculated which model how the listener 40 perceives the source signal S which is depending on the listener's 40 audiogram, i.e. the hearing model 54. For each 1/3 octave frequency band, a band-pass level may be measured, and a gain may be calculated which represent the attenuation of the signal a listener 40 would experience due to their hearing impairment. In some embodiments, the gain per frequency band $H(b)$ may be calculated as indicated below.

$$H(b) = 0, M(b) > M_{max}$$

$$H(b) = HL(b), M(b) < M_{min}$$

$$H(b) = M(b) - \frac{M_{max}}{M_{max} - HL(b)} \big(M(b) - HL(b)\big), M_{min} \leq M(b) \leq M_{max}$$

In the equations presented above, b = 1, ... , B is the index of the 1/3 octave bands, $M(b)$ is the sound pressure in band b and $HL(b)$ is the users audiogram in hearing level, all signals are in dB unit,

There are many hearing models 54 usable with the present disclosure, and the teachings herein are not limited to the models listed. The hearing model 54 may be configured to model hearing impairment that considers either or both conductive and sensorineural hearing loss. Conductive impairment are generally results of e.g. blockage of the ear canal and are manifested as a loudness decrease that equals the degree of impairment. Conductive hearing losses are often treated successfully by medical or surgical means. Sensorineural hearing loss are generally due to decreased sensory ability in the middle ear and, more common, in the inner ear due to a deficiency in the cochlea. In general, this class accounts for most cases of hearing loss. The causes of sensorineural hearing loss are e.g. genetic factors, diseases, high level noise or sound exposure and aging (presbycusis) process.

The hearing model 54 presented above approximates a loudness recruitment hearing loss. A person skilled in the art will, after contemplating the present disclosure, be able to replace and/or expand the hearing model 54 with additional consideration e.g. reduction in amplitude sensitivity, reduced frequency range, loss of dynamic range, loss in spectral details differentiation, and/or any combinations of the afore mentioned.

Several methods exist in the art that are usable to estimate speech intelligibility e.g. Signal-to-Noise Ratio (SNR), Speech Intelligibility Index (SII) or Coherence Signal Intelligibility Index (CSII), Short term objective intelligibility (STOI) etc. Most of the intelligibility methods uses a similarity measure i.e. correlation or coherence between the source signal S (reference) and the processed signal (signal in noise or otherwise deteriorated source signal). The selection of method for intelligibility may impact most of the processing blocks 100, 410, 415, 420, 430, but for a skilled person, this is within the scope of her expertise. In the present disclosure, a correlation-based intelligibility measure is presented, but a person skilled in the art can readily replace the intelligibility measure.

For speech, consider the correlation coefficient in the frequency domain between the source signal S and the hearing-impaired microphone signal 17' as an intelligibility measure. A comparably high correlation generally corresponds to high intelligibility, and a comparably low correlation generally corresponds to low intelligibility. Therefore, a comparably high correlation per frequency band is desired. For music, a correlation-based intelligibility measure is less obvious and may miss components in the objective intelligibility method. However, an SNR based method is intuitively appropriate, and a correlation measure is also suitable since the application herein is not dependent on an absolute value. The processing component may be comprised of frequency selective and adaptive enhancement subcomponents that use e.g. the enhancement parameters for configuration.

In the following, as an exemplary embodiment usable with other teachings of the present disclosure, a desired gain per frequency band will be calculated. The desired gain per frequency band is to be applied to the source signal S using a finite impulse response (FIR) filter with linear or minimum phase. Furthermore, it may also be e.g. a parameterized equalized comprised of a set of infinite impulse response filters. However, the latter may, depending on design, cause a comparably high audio distortion. Hence, the FIR filter is the preferred choice. The following calculation are described for one channel for simplicity, two channels expansion is trivial for the skilled person. For each frame of samples of the source signal $S$ and microphone signals 17', the calculation of new weights per frequency band $W(b)$ is preferably executed. The microphone signals 17' representing sound pressure level per frequency band are denoted $M(b)$ and the source signals S representing sound pressure level per frequency band are denoted $X(b)$. Then, $M(b) = E(b)H(b)W(b)X(b) + H(b)V(b)$, where $E(b)$ is the echo path frequency transfer function, $H(b)$ is the attenuation due to the listener's 40 hearing impairment (comprised in the hearing profile 54), $W(b)$ is the (adaptive) frequency gain applied by the processing block 410 and $V(b)$ is the background noise received by the microphones 17. In matrix form, the same expression would be $M = EHWX + HV$, where $M, X, V$ are vectors where each element corresponds to a band b, $E, H, W$ are diagonal matrices, i.e. all elements are zero except the diagonal. In summary, $E$ is the matrix describing the echo path such that $EX$ equals the source signal $S$ as measured by the microphone 17

As an example on measure of the intelligibility, the sum of the diagonal of the correlation matrix between the source signal and the microphone signal is used i.e. intelligibility is $I = \mathrm{tr}(T_x R_{XM})$ where $R_{XM} = \mathrm{E}(X^T M) = EHWR_{XX} + HR_{XV}$, $R_{XV}$ is the correlation matrix between the source signal and the background noise and $T_x$ is a weight matrix. $R_{XX}$ is the correlation matrix of the source signal $S$, and since it is not normalized, the diagonal element correspond to the sound pressure squared per frequency band. Using this intelligibility measure, the source signal S may be frequency adjusted using $W$ but the $R_{XV}$ is not impacted. In other words, $W$ is a frequency gain matrix which is preferably applied to the source signal S in the processing block 410.

To optimize the selected intelligibility and account for hearing impairment, background noise and sound dose, and since $R_{XV}$ are independent of the weights W, the following optimization may formulated, $w = \arg \max I = \arg \max \mathrm{tr}(T_x R_{XM}) = \arg \max \mathrm{tr}(T_x EHWR_{XX})$.

By adding constrains on weights and amplification, the optimization may be formulated as a second order cone program where the constraints change depending on if the sound pressure shall be limited or not.

If the sound pressure is to be limited:

$$
\begin{aligned}
&\text{maximize} && \mathrm{tr}(T_x EHWR_{XX}) \\
&\text{subjected to} && W \geqslant 0 \\
&\quad 1. && \|XW\|_2 \leq L_{spl} - \|V\|_2 \\
&\quad 2. && \boldsymbol{XW} \geqslant T_v V
\end{aligned}
$$

Or, if the sound pressure is not limited:

$$
\text{maximize} \qquad \mathrm{tr}(T_x EHWR_{XX})
$$

(continued)

| subjected to | $W \geqslant 0$ |
|---|---|
| 1. | $\|W\|_2 \leq \sqrt{B} \cdot \Gamma$ |
| 2. | $XW \geqslant T_v V$ |

The amplification constraint $\Gamma$ is set by configuration, and may be part of the processing parameters and state which level increase is expected when adjusting for the hearing profile 54 of the listener 40. $T_v$, $T_x$ are weight matrices that are used to emphasize some frequency bands over the rest. Typically used in speech application where speech frequencies have different importance for intelligibility. Elements (frequency bands) in the weight matrixes $T_v$, $T_x$ which are to be given focus are set to 1 and all other less important ranges from 1 down to 0. Typically, important frequency bands are 100 - 4000 Hz, 4 - 8 kHz are important for pleasantness and partly for intelligibility, 8 - 12 kHz provide some details but very little intelligibility, and content above 12 kHz are subjected to research.

In some embodiments, a limitation to the maximum gain may be added, $\|W\|_2 \leq \sqrt{B} \cdot \Gamma$ where e.g., $\Gamma = 4$ is equivalent to a maximum of 4 times root mean square amplification.

The frequency gain $W$ may then be converted into a FIR filter using the frequency sampling method. Other design methods or equivalent filter construction are also possible since the desired frequency response of the filter is given by $W$.

Preferably, the adaptive processing block 410 comprises a FIR filter configured to process the frames of the source signal S.

With reference to Fig. 7, a method 300 for controlling a sound dose of a listener 40 according to embodiments of the present disclosure will be presented. The method 300 may be extended to further comprise any suitable feature listed within this disclosure. The method comprises obtaining 310 the previously presented audio profile 50 comprising sound pressure data 52 for the listener 40. The audio profile 50 may be obtained from a remote or local storage 220. If the audio profile 50 is not available, the step of obtaining 310 the audio profile 50 may comprise creating the audio profile 50. The method 300 further comprises obtaining 320 of the current sound pressure $p_c$ experienced by the listener 40. This may be performed by any means disclosed within the present disclosure or any other suitable means usable by the skilled person when implementing the method 300. Based on the sound pressure data 52 and the current sound pressure $p_c$, the method 300 comprises estimating 330 the current estimated time period $T_{ce}$. The current estimated time period $T_{ce}$ describe the estimated time until the sound dose $D$ of the listener 40 exceed a sound dose threshold $L$. Typically, as is taught within the present disclosure, there may me be more than one sound dose threshold $L$ used. Each sound dose threshold $L$ may be associated with one separate dose period $T_D$ and/or one separate set of sound pressure data 52. As taught, the estimating 330 of the current estimated time period $T_{ce}$ may optionally be based on one or more prediction models 56 and the estimating 330 may comprise determining 335 the prediction model 56.

If the current estimated time period $T_{ce}$ is below the dose period $T_D$ associated with the sound dose threshold $L$, the method 300 further comprises, based on the sound pressure data 52 and the current sound pressure $p_c$, calculating 340 an updated sound pressure $p_u$. As is described within the present disclosure, the updated sound pressure $p_u$ is a sound pressure for which the current updated time period $T_{cu}$ meets or exceeds the dose period ($T_D$). The method 300 may further comprise providing 350 instructions 60 for controlling the current sound pressure $p_c$ to meet the updated sound pressure $p_{cu}$.

It should be mentioned that the instructions 60 for controlling the current sound pressure $p_c$ to meet the updated sound pressure $p_{cu}$ may be any suitable instructions 60 usable directly by an audio playback arrangement 10 to reduce a signal level provided to the transducer 15. In one embodiment, the instructions 60 are preferably provided to the audio playback arrangement 10, and particularly to the controller 100 of the audio playback arrangement 10. Additionally, or alternatively, the instructions 60 may be indications or urges for the listener to reduce the playback volume of the audio playback arrangement 10. The instructions 10 may be audible instructions playable by the audio playback arrangement 10 and/or visual instructions presented by e.g. the portable electronic equipment 30.

The obtained audio profile 50 and sound pressure data 52 usable in the method 300 may be in any suitable shape or form and may be according to any embodiment or feature presented within the present disclosure.

In some embodiments of the method 300, the audio profile 50 may comprise the hearing profile 54 of the listener 40. In such embodiments, the current estimated time $T_{ce}$ may further be based on the hearing profile 54 by frequency weighting 325 the current sound pressure $p_u$ based on the hearing profile 54 of the listener 40. The frequency weighting 325 may be performed as part of obtaining 320 of the current sound pressure $p_c$ experienced by the listener 40.

The method may optionally comprise updating 360 the sound pressure data 52 of the audio profile 50 for the listener 40 based on the current sound pressure $p_c$.

In Figs. 8a-e, the controller 100 for controlling an audio playback arrangement 10 is shown. The controller 100 may be configured to cause e.g. the execution of the method 300 described with reference to Fig. 7. Generally, the controller 100, according to any embodiment, may be configured to cause performance of (e.g., perform) one or more steps of the method 300 described in connection with Fig. 7. This means that e.g. actual obtaining, provisioning, calculation etc., may not be performed by the controller 100; the controller may be configured to cause other devices, modules or blocks to perform the actual operations. However, the controller 100 may very well be configured to perform one or more steps of the method 300 described in connection with Fig. 7. In a preferred embodiment, the controller 100 is configured to perform one or more steps of the method 300 described in connection with Fig. 7 and configured to cause performance of the remaining steps of the method 300 described with reference to Fig. 7.

For example, as shown in Fig. 8b, the controller 100 may be comprisable, e.g. comprised, in a first apparatus 10. The first apparatus may be an audio playback arrangement 10, e.g. any of the audio playback arrangements 10 presented with reference to Figs. 3a-d.

Alternatively, or additionally, the controller 100 may, as shown in Fig. 8c be comprisable, e.g. comprised, in the electronic device 30. The electronic device 30 may be the portable electronic device 30 of Fig. 3b, Fig. 4 and/or the rendering device 30 as described with reference to Fig. 5. The electronic device 30 may be any electronic device 30 suitable for controlling the sound dose D of the listener 40.

Alternatively, or additionally, the controller 100 may, as shown in Fig. 8d be comprisable, e.g. comprised, in the server 210 or cloud server 210. The cloud server 210 may be the cloud server 210 of Fig. 4. The cloud server 210 may be any server 210 suitable for controlling the sound dose $D$ of the listener 40.

Alternatively, or additionally, the controller 100 may be comprisable, e.g. comprised, in a system 200. The system 200 may be any system 200 suitable for controlling the sound dose $D$ of the listener 40, e.g. the system 200 presented with reference to Figs. 4 and 5.

As the skilled person will appreciate after contemplating the teachings of the present disclosure, the described embodiments and their equivalents may be realized in software or hardware or a combination thereof. The embodiments may be performed by general purpose circuitry. Examples of general purpose circuitry include digital signal processors (DSP), central processing units (CPU), co-processor units, field programmable gate arrays (FPGA) and other programmable hardware. Alternatively, or additionally, the embodiments may be performed by specialized circuitry, such as application specific integrated circuits (ASIC). The general purpose circuitry and/or the specialized circuitry may, for example, be associated with or comprised in an audio arrangement 10.

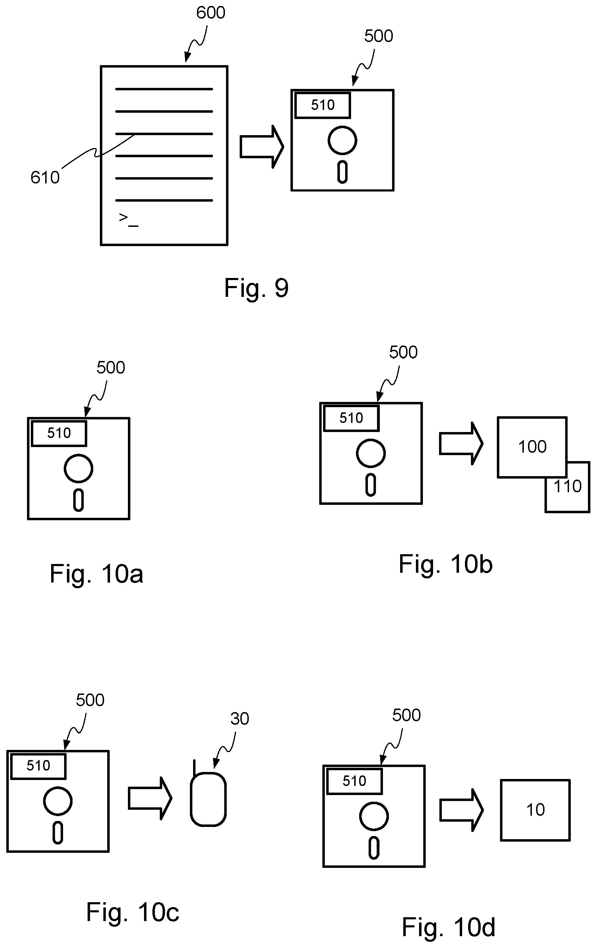

According to some embodiments, as illustrated in Fig. 8, a computer program 600 is stored onto a non-transitory computer readable medium 510 to form a computer program product 500. The computer program product 500 comprises one or more non-transitory computer readable medium 510 and one or more computer programs 600. The non-transitory computer readable medium may be any suitable non-transitory computer readable exemplified but not limited by, a universal serial bus (USB) memory, compact disc (CD) ROM, a plug-in card, an embedded drive, or a read only memory (ROM). Fig. 9a illustrates an example computer readable medium in the form of a vintage 5,25-inch floppy disc 510. The computer readable medium 510 has stored thereon the computer program 600 comprising program instructions 610. The computer program 600 is loadable into a controller 100, see Fig. 9b, which may, for example, be comprised in an electronic device, see Fig. 9c, and/or in an audio playback arrangement 10, see Fig. 9d. When loaded into controller 100, the computer program 600 may be stored in a memory 110 associated with, or comprised in, the controller 100. According to some embodiments, the computer program 600 may, when loaded into, and run by, the controller 100, cause execution of method steps according to, for example, the method 300 illustrated in Fig. 7, or otherwise described herein.

Modifications and other variants of the described embodiments will come to mind to one skilled in the art having benefit of the teachings presented in the foregoing description and associated drawings. Therefore, it is to be understood that the embodiments are not limited to the specific example embodiments described in this disclosure and that the scope of protection is solely defined by the appended claims.

Furthermore, although specific terms may be employed herein, they are used in a generic and descriptive sense only and not for purposes of limitation.

In addition, singular references do not exclude a plurality. Finally, reference signs in the claims are provided merely as a clarifying example and should not be construed as limiting the scope of the claims in any way.

**Claims**

1.   A system (200) for controlling a sound dose (*D*) of a listener (40), wherein the sound dose *(D)* describes a sliding average of a current sound pressure ($p_c$) squared over a dose period ($T_D$), the system (200) comprising:

a cloud server (210) operatively connected to a remote storage (220) configured to store a plurality of audio profiles (50), each comprising sound pressure data (52) and a hearing profile (54) associated with specific users (40) of the system (200),
one or more audio playback arrangements (10) comprising a transducer (15), a controller (100), and a microphone (17) arranged to measure the current sound pressure ($p_c$) experienced by the listener (40), wherein said one or more audio playback arrangements (10) are operatively connected to the cloud server (210) and configured to determine an identity of the listener (40), and the system (200) is configured to associate said identity with a specific user (40) of the system (200),

the cloud server (210) and/or the controller (100) of said one or more audio playback arrangements (10) comprises controlling circuitry configured to cause:

> obtaining (310) of an audio profile (50) comprising sound pressure data (52) and a hearing profile (54) for the listener (40),
> obtaining (320) of a current sound pressure ($p_c$) experienced by the listener (40),
> based on the sound pressure data (52), the hearing profile (54) of the listener (40) and the current sound pressure ($p_c$), estimating (330) of a current estimated time period ($T_{ce}$) describing an estimated time until the sound dose *(D)* of the listener (40) exceeds a sound dose threshold (L) associated with the dose period ($T_D$), by frequency weighting (325) the current sound pressure ($p_c$) based on the hearing profile (54) of the listener (40),
> responsive to the current estimated time period ($T_{ce}$) being below a dose period ($T_D$) associated with the sound dose threshold (L):
>
>> based on the sound pressure data (52) and the current sound pressure ($p_c$), calculating (340) of an updated sound pressure ($p_u$), for which a current updated time period ($T_{cu}$), describing an estimated time until the sound dose *(D)* of the listener (40) exceeds the sound dose threshold (L), meets or exceeds the dose period ($T_D$), and
>> providing (350) of instructions (60) for controlling the current sound pressure ($p_c$) to meet the updated sound pressure ($p_{cu}$).

2. The system (200) of claim 1, wherein the sound pressure data (52) of the audio profile (50) comprises historic sound pressure data (52) comprising previous sound pressure experienced by the listener (40).

3. The system (200) of claim 1 or 2, wherein the sound pressure data (52) of the audio profile (50) comprises an accumulated sound pressure (52) experienced by the listener (40).

4. The system (200) of any one of the preceding claims, wherein the sound pressure data (52) of the audio profile (50) comprises a sound pressure trend experienced by the listener (40).

5. The system (200) of any one of the preceding claims, wherein the current sound pressure ($p_c$) is obtained (320) from a microphone (17) arranged at an ear (42, 44) of the listener (40).

6. The system (200) of any one of the preceding claims, wherein estimating (330) a current estimated time period ($T_{ce}$) describing the estimated time until the sound dose *(D)* of the listener (40) exceed the sound dose threshold *(L)* comprises using (335) a prediction model (56).

7. The system (200) of claim 6, wherein the prediction model (56) comprises an average model.

8. The system (200) of claim 6 or 7, wherein the prediction model (56) comprises a Recurring Neural Network, RNN, model.

9. The system (200) of any one of the preceding claims, wherein the controlling circuitry is further configured to cause updating (360) of the sound pressure data (52) of the audio profile (50) for the listener (40) based on the current sound pressure ($p_c$).

10. The system (200) of any one of the preceding claims, wherein the controlling circuitry is configured to cause providing (350) of instructions (60) for controlling the current sound pressure (pc) for a left ear (42) and/or a right ear (44) of the listener (40) and the audio profile (50) comprises separate sound pressure data (52) for each of the left ear (42) and the right ear (44) of the listener (40).

**Patentansprüche**

1. System (200) zum Steuern einer Schalldosis (*D*) eines Hörers (40), wobei die Schalldosis *(D)* einen gleitenden Mittelwert eines aktuellen Schalldrucks ($p_c$) quadriert über eine Dosisperiode ($T_D$) beschreibt, wobei das System (200) Folgendes umfasst:

   einen Cloud-Server (210), der mit einem entfernten Speicher (220) wirkverbunden ist, der so konfiguriert ist, dass er eine Vielzahl von Audioprofilen (50) speichert, von denen jedes Schalldruckdaten (52) und ein Hörprofil (54) umfasst, die bestimmten Benutzern (40) des Systems (200) zugeordnet sind,
   eine oder mehrere Audiowiedergabeanordnungen (10), umfassend einen Wandler (15), eine Steuerung (100) und ein Mikrofon (17), die so angeordnet sind, dass sie den aktuellen Schalldruck ($p_c$) messen, dem der Hörer (40) ausgesetzt ist, wobei die eine oder mehrere Audiowiedergabeanordnungen (10) mit dem Cloud-Server (210) wirkverbunden und so konfiguriert sind, dass sie eine Identität des Hörers (40) bestimmen, und das System (200) so konfiguriert ist, dass es die Identität einem bestimmten Benutzer (40) des Systems (200) zuordnet,
   der Cloud-Server (210) und/oder die Steuerung (100) der einen oder mehreren Audiowiedergabeanordnungen (10) eine Steuerschaltung umfasst, die so konfiguriert ist, dass sie Folgendes veranlasst:

   Erhalten (310) eines Audioprofils (50), umfassend Schalldruckdaten (52) und ein Hörprofil (54) für den Hörer (40),
   Erhalten (320) eines aktuellen Schalldrucks ($p_c$), dem der Hörer (40) ausgesetzt ist,
   anhand der Schalldruckdaten (52), dem Hörprofil (54) des Hörers (40) und dem aktuellen Schalldruck ($p_c$), Schätzen (330) eines aktuellen geschätzten Zeitraums ($T_{ce}$), der eine geschätzte Zeit beschreibt, bis die Schalldosis (*D*) des Hörers (40) eine Schalldosisschwelle *(L)* überschreitet, die der Dosisperiode ($T_D$) zugeordnet ist, durch Frequenzbewertung (325) des aktuellen Schalldrucks ($p_c$) anhand des Hörprofils (54) des Hörers (40),
   als Reaktion auf den aktuellen geschätzten Zeitraum ($T_{ce}$), der unter einer Dosisperiode ($T_D$) liegt, der der Schalldosisschwelle *(L)* zugeordnet ist:

   anhand der Schalldruckdaten (52) und des aktuellen Schalldrucks ($p_c$), Berechnen (340) eines aktualisierten Schalldrucks ($p_u$), für den ein aktueller aktualisierter Zeitraum ($T_{cu}$), der eine geschätzte Zeit beschreibt, bis die Schalldosis (D) des Hörers (40) die Schalldosisschwelle *(L)* überschreitet, die Dosisperiode ($T_D$) erreicht oder überschreitet, und
   Bereitstellen (350) von Anweisungen (60) zum Steuern des aktuellen Schalldrucks ($p_c$), um den aktualisierten Schalldruck ($p_{cu}$) zu erreichen.

2. System (200) nach Anspruch 1, wobei die Schalldruckdaten (52) des Audioprofils (50) historische Schalldruckdaten (52) umfassen, umfassend den vorherigen Schalldruck, dem der Hörer (40) ausgesetzt ist.

3. System (200) nach Anspruch 1 oder 2, wobei die Schalldruckdaten (52) des Audioprofils (50) einen akkumulierten Schalldruck (52) umfassen, dem der Hörer (40) ausgesetzt ist.

4. System (200) nach einem der vorhergehenden Ansprüche, wobei die Schalldruckdaten (52) des Audioprofils (50) einen Schalldruckverlauf umfassen, dem der Hörer (40) ausgesetzt ist.

5. System (200) nach einem der vorhergehenden Ansprüche, wobei der aktuelle Schalldruck ($p_c$) von einem Mikrofon (17) erhalten wird (320), das an einem Ohr (42, 44) des Hörers (40) angeordnet ist.

6. System (200) nach einem der vorhergehenden Ansprüche, wobei das Schätzen (330) eines aktuellen geschätzten Zeitraums ($T_{ce}$), der die geschätzte Zeit beschreibt, bis die Schalldosis (*D*) des Hörers (40) die Schalldosisschwelle *(L)* überschreitet, die Verwendung (335) eines Vorhersagemodells (56) umfasst.

7. System (200) nach Anspruch 6, wobei das Vorhersagemodell (56) ein Durchschnittsmodell umfasst.

8. System (200) nach Anspruch 6 oder 7, wobei das Vorhersagemodell (56) ein Recurring-Neural-Network-, RNN-, Modell umfasst.

9. System (200) nach einem der vorhergehenden Ansprüche, wobei die Steuerschaltung ferner so konfiguriert ist, dass sie ein Aktualisieren (360) der Schalldruckdaten (52) des Audioprofils (50) für den Hörer (40) anhand des aktuellen Schalldrucks ($p_c$) veranlasst.

10. System (200) nach einem der vorhergehenden Ansprüche, wobei die Steuerschaltung so konfiguriert ist, dass sie das Bereitstellen (350) von Anweisungen (60) zum Steuern des aktuellen Schalldrucks ($p_c$) für ein linkes Ohr (42) und/oder ein rechtes Ohr (44) des Hörers (40) veranlasst und das Audioprofil (50) separate Schalldruckdaten (52) für jeweils das linke Ohr (42) und das rechte Ohr (44) des Hörers (40) umfasst.

## Revendications

1. Système (200) pour commander une dose sonore (D) d'une personne qui écoute (40), dans lequel la dose sonore (D) décrit une moyenne mobile d'une pression sonore courante ($p_c$) au carré sur une période de dose ($T_D$), le système (200) comprenant :

   un serveur cloud (210) connecté de manière fonctionnelle à une mémoire distante (220) configurée pour stocker une pluralité de profils audio (50), comprenant chacun des données de pression sonore (52) et un profil auditif (54) associé à des utilisateurs (40) spécifiques du système (200),
   un ou plusieurs agencements de lecture audio (10) comprenant un transducteur (15), un dispositif de commande (100), et un microphone (17) agencé pour mesurer la pression sonore courante ($p_c$) ressentie par la personne qui écoute (40), dans lequel lesdits un ou plusieurs agencements de lecture audio (10) sont connectés de manière fonctionnelle au serveur cloud (210) et configurés pour déterminer une identité de la personne qui écoute (40), et le système (200) est configuré pour associer ladite identité à un utilisateur (40) spécifique du système (200), le serveur cloud (210) et/ou le dispositif de commande (100) desdits un ou plusieurs agencements de lecture audio (10) comprennent un ensemble de circuits de commande configuré pour provoquer :

   l'obtention (310) d'un profil audio (50) comprenant des données de pression sonore (52) et un profil auditif (54) pour la personne qui écoute (40),
   l'obtention (320) d'une pression sonore courante ($p_c$) ressentie par la personne qui écoute (40),
   sur la base des données de pression sonore (52), du profil auditif (54) de la personne qui écoute (40) et de la pression sonore courante ($p_c$), l'estimation (330) d'une période de temps estimée courante ($T_{ce}$) décrivant un temps estimé jusqu'à ce que la dose sonore (D) de la personne qui écoute (40) dépasse un seuil de dose sonore (L) associé à la période de dose ($T_D$), par pondération de fréquence (325) de la pression sonore courante ($p_c$) sur la base du profil auditif (54) de la personne qui écoute (40),
   en réponse à la période de temps estimée courante ($T_{ce}$) qui est inférieure à une période de dose ($T_D$) associée au seuil de dose sonore (L) :

   sur la base des données de pression sonore (52) et de la pression sonore courante ($p_c$), le calcul (340) d'une pression sonore mise à jour ($p_u$), pour laquelle une période de temps mise à jour courante ($T_{cu}$), décrivant un temps estimé jusqu'à ce que la dose sonore (D) de la personne qui écoute (40) dépasse le seuil de dose sonore (L), atteint ou dépasse la période de dose ($T_D$), et
   la fourniture (350) d'instructions (60) pour commander la pression sonore courante ($p_c$) pour qu'elle atteigne la pression sonore mise à jour ($p_{cu}$).

2. Système (200) selon la revendication 1, dans lequel les données de pression sonore (52) du profil audio (50) comprennent des données de pression sonore (52) historiques comprenant une pression sonore précédente ressentie par la personne qui écoute (40).

3. Système (200) selon la revendication 1 ou 2, dans lequel les données de pression sonore (52) du profil audio (50) comprennent une pression sonore (52) accumulée ressentie par la personne qui écoute (40).

4. Système (200) selon l'une quelconque des revendications précédentes, dans lequel les données de pression sonore (52) du profil audio (50) comprennent une tendance de pression sonore ressentie par la personne qui écoute (40).

5. Système (200) selon l'une quelconque des revendications précédentes, dans lequel la pression sonore courante ($p_c$) est obtenue (320) à partir d'un microphone (17) agencé au niveau d'une oreille (42, 44) de la personne qui écoute (40).

6. Système (200) selon l'une quelconque des revendications précédentes, dans lequel l'estimation (330) d'une période de temps estimée courante ($T_{ce}$) décrivant le temps estimé jusqu'à ce que la dose sonore (D) de la personne qui écoute (40) dépasse le seuil de dose sonore ($L$) comprend l'utilisation (335) d'un modèle de prédiction (56).

**7.** Système (200) selon la revendication 6, dans lequel le modèle de prédiction (56) comprend un modèle moyen.

**8.** Système (200) selon la revendication 6 ou 7, dans lequel le modèle de prédiction (56) comprend un modèle de réseau neuronal récurrent, RNN.

**9.** Système (200) selon l'une quelconque des revendications précédentes, dans lequel l'ensemble de circuits de commande est en outre configuré pour provoquer la mise à jour (360) des données de pression sonore (52) du profil audio (50) pour la personne qui écoute (40) sur la base de la pression sonore courante ($p_c$).

**10.** Système (200) selon l'une quelconque des revendications précédentes, dans lequel l'ensemble de circuits de commande est configuré pour provoquer la fourniture (350) d'instructions (60) pour commander la pression sonore courante (pc) pour une oreille gauche (42) et/ou une oreille droite (44) de la personne qui écoute (40) et le profil audio (50) comprend des données de pression sonore (52) distinctes pour chacune de l'oreille gauche (42) et de l'oreille droite (44) de la personne qui écoute (40).

Fig. 1

Fig. 2a

Fig. 2b

Fig. 3a

Fig. 3b

Fig. 3c

Fig. 3d

Fig. 4

Fig. 5

Fig. 6

Fig. 7

Fig. 8a    Fig. 8b

Fig. 8c    Fig. 8d

Fig. 8e

Fig. 9

Fig. 10a

Fig. 10b

Fig. 10c

Fig. 10d

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2016316306 A1
- US 2008159547 A1
- US 2008015463 A1